# EUROPEAN PATENT APPLICATION

(11) **EP 2 336 358 A1**
(43) Date of publication of application: **22.06.2011**
(21) Application number: 10189426.9
(22) Date of filing: 06.05.2008
(51) Int. Cl.: C12Q 1/68

(54) **Simultaneous detection of multiple nucleic acid sequences in a reaction**

(62) Divisional of application: 08155687.0
(71) Applicant: QIAGEN GmbH, 40724 Hilden (DE); Qiagen Hamburg GmbH, 22767 Hamburg (DE)
(72) Inventor: Rothmann, Thomas, 40724 Hilden (DE); Engel, Holger, 40724 Hilden (DE); Laue, Thomas, 27404 Elsdorf (DE)
(74) Representative: Vossius & Partner

(57) **Abstract**

The present invention relates to a method for simultaneously amplifying and detecting nucleic acid sequences in a reaction comprising the following steps, (i) providing a sample comprising at least one nucleic acid molecule, (ii) providing reagents for performing an amplification reaction, wherein the reagents comprise at least three probe sets, wherein (a) each probe set consists of at least three probes, (b) each of the probes is specific for a nucleic acid sequence, (c) each of the probes in a given probe set carries a different label, (d) all of the probes in a given probe set have a similar, preferably identical melting temperature (Tₘ) when they are dissociated from their target nucleic acid sequence by heating, (iii) amplifying the nucleic acid sequences in the reaction, (iv) detecting the amplified nucleic acids by determining whether the labeled probe has bound its nucleic acid sequence, and (v) detecting the temperature at which each given labeled probe dissociates from the nucleic acid sequence to which it has bound.

## Description

### FIELD OF THE INVENTION

The present invention is in the field of biology and chemistry, more in particular in the field of molecular biology and human genetics. The invention relates to the field of identifying certain nucleic acid sequences in a sample. Particularly, the invention is in the field of simultaneously amplifying and detecting nucleic acid sequences in a reaction. The invention relates to methods, kits, and systems for detection of nucleic acid sequences in a sample.

### BACKGROUND OF THE INVENTION

Diagnostic assays that sensitively, specifically, and quickly detect biological agents, e.g., pathogens, in samples are becoming increasingly important for both disease and diagnostic bio agent monitoring. Few assays are able to accurately detect physiologically or clinically relevant organisms on an appropriate time scale for the early detection of the presence of an infective or otherwise harmful agent.

A DNA microarray is a collection of microscopic DNA spots, commonly representing single genes, arrayed on a solid surface by covalent attachment to a chemical matrix. DNA arrays are different from other types of microarray only in that they either measure DNA or use DNA as part of its detection system. Qualitative or quantitative measurements with DNA microarrays utilize the selective nature of DNA-DNA or DNA-RNA hybridization under high-stringency conditions and fluorophore-based detection. DNA arrays are commonly used for expression profiling, *i.e.*, monitoring expression levels of thousands of genes simultaneously, or for comparative genomic hybridization. The drawback with this system is that multiple steps need to be performed prior to analysis. Also, the array is not sensitive.

To date, the most sensitive detection methods involve PCR. Determining the presence or absence of a plurality of biological agents in a single sample can be performed using multiplexed detection methods.

Multiplex PCR uses multiple, unique primer sets within a single PCR reaction to produce amplicons of varying sizes specific to different DNA sequences, *i.e.* different transgenes. By targeting multiple genes at once, additional information may be gained from a single test run that otherwise would require several times the reagents and more time to perform. Annealing temperatures for each of the primer sets must be optimized to work correctly within a single reaction, and amplicon sizes, *i.e.*, their base pair length, should be different enough to form distinct bands when visualized by gel electrophoresis.

Multiplexed real time PCR is one method that can be used for a diagnostic assay. Assays based on PCR can be limited by the complexity of optimizing the PCR reactions to test for multiple agents in a cost-effective number of reaction tubes. As a general rule, the number of probes needed to support a highly specific confirmation result range from two to as many as six sequences. As one of skill in the art will be aware, optimizing a PCR reaction with many different primer pairs and probes can be a formidable task that becomes increasingly unmanageable as the number of targets to be detected increases. Assays based on PCR can also be limited by the number of unique labels available for analysis of results. For example, real time PCR assays generally employ fluorescent labels.

The number of labels that can be used in a single reaction is limited by the number of fluorescent color channels available in the optical detection system used.

It would be advantageous to have a method for simultaneously amplifying and detecting multiple nucleic acid sequences in one container.

WO 2005/111243 A2 relates to a method of detecting agents in two containers. The drawback of the method disclosed in WO 2005/111243 A2 is the fact that two containers are necessary. Ideally, the reaction would require one container.

### SUMMARY OF THE INTENTION

The present invention relates to a method for simultaneously amplifying and detecting nucleic acid sequences in a reaction comprising the following steps, (i) providing a sample comprising at least one nucleic acid molecule, (ii) providing reagents for performing an amplification reaction, wherein the reagents comprise at least three probe sets, wherein (a) each probe set consists of at least three probes, (b) each of the probes is specific for a nucleic acid sequence, (c) each of the probes in a given probe set carries a different label, (d) all of the probes in a given probe set have a similar, preferably identical melting temperature (Tₘ) when they are dissociated from their target nucleic acid sequence by heating, (iii) amplifying the nucleic acid sequences in the reaction, (iv) detecting the amplified nucleic acids by determining whether the labeled probe has bound its nucleic acid sequence, and (v) detecting the temperature at which each given labeled probe dissociates from the nucleic acid sequence to which it has bound.

As used herein the term "nucleic acid sequence" is, in the context of the present invention, a sequence on a nucleic acid. A nucleic acid may be, *inter alia,* RNA, DNA, cDNA (complementary DNA), LNA (locked nucleic acid), mRNA (messenger RNA), mtRNA (mitochondrial), rRNA (ribosomal RNA), tRNA (transfer RNA), nRNA (nuclear RNA), siRNA (short interfering RNA), snRNA (small nuclear RNA), snoRNA (small nucleolar RNA), scaRNA (Small Cajal Body specific RNA), microRNA, dsRNA (doubled-stranded RNA), ribozyme, riboswitch, viral RNA, dsDNA (double-stranded DNA), ssDNA (single-stranded DNA), plasmid DNA, cosmid DNA, chromosomal DNA, viral DNA, mtDNA (mitochondrial DNA), nDNA (nuclear DNA), snDNA (small nuclear DNA) or the like or any other class or sub-class of nucleic acid which is distinguishable from the bulk nucleic acid in a sample.

As used herein the term "probe" is a nucleic acid which is able to bind another nucleic acid.

As used herein the term "tissue" refers to any tissue or fluid in a human, animal or plant including, but not limited to breast, prostate, blood, serum, cerebrospinal fluid, liver, kidney, breast, head and neck, pharynx, thyroid, pancreas, stomach, colon, colorectal, uterus, cervix, bone, bone marrow, testes, brain, neural tissue, ovary, skin, and lung.

As used herein the term "probe set" is a set of three or more agents that may interact with a nucleic acid molecule at a specific position, *i.e.* sequence.

Herein, a "label" is a moiety that is bound covalently or non-covalently to a probe where it can give rise to signal which may be detected by optical or other physical means.

### DETAILED DESCRIPTION OF THE INVENTION

The present invention relates to a method for simultaneously amplifying and detecting nucleic acid sequences in a reaction comprising the following steps, (i) providing a sample comprising at least one nucleic acid molecule, (ii) providing reagents for performing an amplification reaction, wherein the reagents comprise at least two probe sets, wherein (a) each probe set consists of at least three probes, (b) each of the probes is specific for a nucleic acid sequence, (c) each of the probes in a given probe set carries a different label, (d) all of the probes in a given probe set have a similar, preferably identical melting temperature (Tₘ) when they are dissociated from their target nucleic acid sequence by heating, (iii) amplifying the nucleic acid sequences in the reaction, (iv) detecting the amplified nucleic acids by determining whether the labeled probe has bound its nucleic acid sequence, and (v) detecting the temperature at which each given labeled probe dissociates from the nucleic acid sequence to which it has bound.

In further embodiment the method comprises at least three probe sets, wherein each probe set consists of at least three probes.

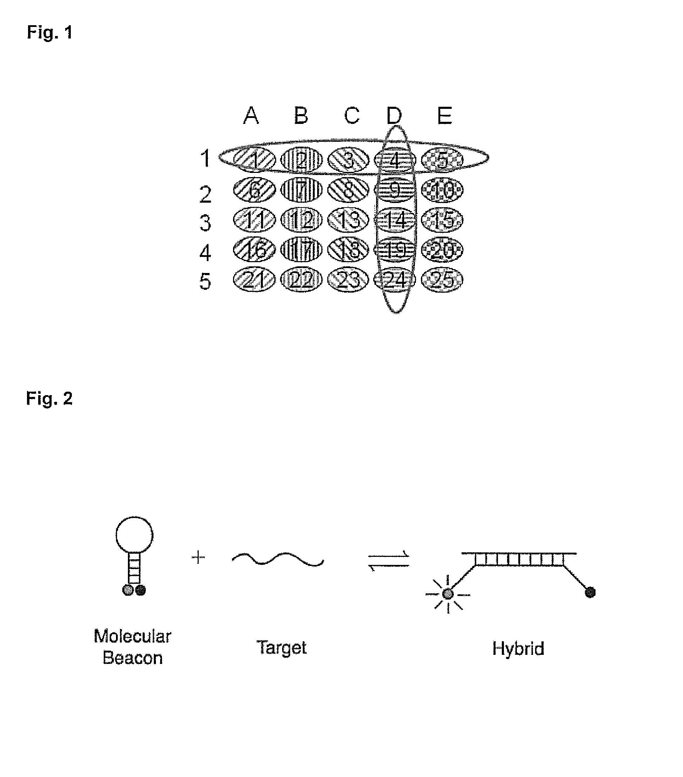

In order to better elucidate the invention we point to figure 1. A probe set according to the invention comprises at least 3 probes. A probe set may seen as all those probes that share a common label but also as all those probes that share a common melting temperature (Tₘ). Ideally, the probes in a probe set that have the same label or labels that are not distinguishable from one another have different melting temperatures. The probes in a probe set that have identical or very similar melting temperatures should have different labels.

The person skilled in the art will know that the reagents will, ordinarily, comprise for example an enzyme for amplification, a buffer, nucleotides and the like. This of course depends on the type of amplification.

The inventors have developed a method which makes it possible to perform a multiplex amplification reaction with, for example 20 templates. In one embodiment 5 different labels are used and all the probes that share a common label have a slightly varying melting temperature. All the probes that share a common melting temperature on the other hand have a different label. By detecting the label and the melting temperature either during or after amplification the inventors have for the first time provided for a means which makes it possible to analyze, e.g. said 20 templates in one tube.

In order to further elucidate the general principle please see Fig. 1. Here, all the probes in row 1 share a common label. All the probes in row one differ with respect to their melting temperature. All the probes in column D share a given melting temperature. Given a scenario in which the probes in row one have a green label and the probes in column D, which all differ with respect to their label share 55°C as a common melting temperature, it would be possible to determine whether a template for probe "4" is present in the reaction because this is the case if a signal is detected from the green label at 55°C melting temperature.

In principle this "detecting the amplified nucleic acids by determining whether the labeled probe has bound its nucleic acid sequence", and "detecting the temperature at which each given labeled probe dissociates from the nucleic acid sequence to which it has bound" may be done at the end of given reaction or during the reaction.

Various amplification methods may be applied, these are for example, rolling circle amplification (such as in Liu, et al., "Rolling circle DNA synthesis: Small circular oligonucleotides as efficient templates for DNA polymerases," J. Am. Chem. Soc. 118:1587-1594 (1996).), isothermal amplification (such as in Walker, et al., "Strand displacement amplification-an isothermal, in vitro DNA amplification technique", Nucleic Acids Res. 20(7):1691-6 (1992)), ligase chain reaction (such as in Landegren, et al., "A Ligase-Mediated Gene Detection Technique," Science 241:1077-1080, 1988, or, in Wiedmann, et al., "Ligase Chain Reaction (LCR)-Overview and Applications," PCR Methods and Applications (Cold Spring Harbor Laboratory Press, Cold Spring Harbor Laboratory, NY, 1994) pp. S51-S64.). Polymerase chain reaction amplification is, however, preferred.

If the reaction is a polymerase chain reaction the "detecting the amplified nucleic acids by determining whether the labeled probe has bound its nucleic acid sequence", and "detecting the temperature at which each given labeled probe dissociates from the nucleic acid sequence to which it has bound" may be done after each cycle, after one cycle, after more than one cycle, in intervals, or at the end of the complete PCR reaction.

A PCR reaction may consist of 10 to 100 "cycles" of denaturation and synthesis of a DNA molecule. In a preferred embodiment, the temperature at which denaturation is done in a thermocycling amplification reaction is between about 90°C to greater than 95°C, more preferably between 92-94°C. Preferred thermo cycling amplification methods include polymerase chain reactions involving from about 10 to about 100 cycles, more preferably from about 25 to about 50 cycles, and peak temperatures of from about 90°C to greater than 95°C, more preferably 92-94°C. In a preferred embodiment, a PCR reaction is done using a DNA Polymerase I to produce, in exponential quantities relative to the number of reaction steps involved, at least one target nucleic acid sequence, given (a) that the ends of the target sequence are known in sufficient detail that oligonucleotide primers can be synthesized which will hybridize to them and (b) that a small amount of the target sequence is available to initiate the chain reaction. The product of the chain reaction will be a discrete nucleic acid duplex with termini corresponding to the ends of the specific primers employed. Any source of nucleic acid, in purified or non-purified form, can be utilized as the starting nucleic acid, if it contains or is thought to contain the target nucleic acid sequence desired. Thus, the process may employ, for example, DNA which may be single stranded or double stranded. In addition, a DNA-RNA hybrid which contains one strand of each may be utilized. A mixture of any of these nucleic acids may also be employed, or the nucleic acids produced from a previous amplification reaction using the same or different primers may be so utilized. The nucleic acid amplified is preferably DNA, The target nucleic acid sequence to be amplified may be only a fraction of a larger molecule or can be present initially as a discrete molecule, so that the target sequence constitutes the entire nucleic acid. It is not necessary that the target sequence to be amplified be present initially in a pure form; it may be a minor fraction of a complex mixture or a portion of nucleic acid sequence due to a particular animal which organism might constitute only a very minor fraction of a particular biological sample. The starting nucleic acid may contain more than one desired target nucleic acid sequence which may be the same or different. Therefore, the method is useful for amplifying simultaneously multiple target nucleic acid sequences located on the same or different nucleic acid molecules. The nucleic acid(s) may be obtained from any source and include plasmids and cloned DNA, DNA from any source, including bacteria, yeast, viruses, and higher organisms such as plants or animals. DNA may be extracted from, for example, blood or other fluid, or tissue material such as chorionic villi or amniotic cells by a variety of techniques such as that described by Maniatis et al., Molecular Cloning: A Laboratory Manual, (New York: Cold Spring Harbor Laboratory) pp 280-281 (1982). Additionally the Templex technology may be applied which combines Genaco's Tem-PCR technology and Luminex's xMAP technology.

The assay makes use of locus-specific primers. Oligonucleotide primers may be prepared using any suitable method, such as, for example, the phosphotriester and phosphodiester methods or automated embodiments thereof. In one such automated embodiment diethylophosphoramidites are used as starting materials and may be synthesized as described by Beaucage et al., Tetrahedron Letters, 22:1859-1862 (1981), which is hereby incorporated by reference. One method for synthesizing oligonucleotides on a modified solid support is described in U.S. Pat. No. 4,458,006, which is hereby incorporated by reference. It is also possible to use a primer which has been isolated from a biological source (such as a restriction endonuclease digest). Preferred primers have a length of from about 15-100, more preferably about 20-50, most preferably about 20-40 bases. It is essential that the primers of the method span the region comprising the target sequence. The target nucleic acid sequence is amplified by using the nucleic acid containing that sequence as a template. [f the nucleic acid contains two strands, it is necessary to separate the strands of the nucleic acid before it can be used as the template, either as a separate step or simultaneously with the synthesis of the primer extension products. This strand separation can be accomplished by any suitable denaturing method including physical, chemical, or enzymatic means. One physical method of separating the strands of the nucleic acid involves heating the nucleic acid until it is completely (>99%) denatured. Typical heat denaturation may involve temperatures ranging from about 80°C to 105°C, preferably about 90°C to about 98°C, still more preferably 93°C to 95°C, for times ranging from about 1 to 10 minutes. In the case of isothermal amplification the strand separation may also be induced by an enzyme from the class of enzymes known as helicases or the enzyme RecA, which has helicase activity and is known to denature DNA. The reaction conditions suitable for separating the strands of nucleic acids with helicases are described by Cold Spring Harbor Symposia on Quantitative Biology, Vol. XLIII "DNA: Replication and Recombination" (New York: Cold Spring Harbor Laboratory, 1978), and techniques for using RecA are reviewed in C. Radding, Ann. Rev. Genetics, 16:405-37 (1982), which is hereby incorporated by reference.

This synthesis can be performed using any suitable method. Generally, it occurs in a buffered aqueous solution. In some preferred embodiments, the buffer pH is about 7.5-8.9. Preferably, a molar excess (for cloned nucleic acid, usually about 1000:1 primer:template, and for genomic nucleic acid, usually about 106 :1 primer:template) of the oligonucleotide primers is added to the buffer containing the separated template strands. It is understood, however, that the amount of complementary strand may not be known if the process herein is used for some applications, so that the amount of primer relative to the amount of complementary strand cannot be determined with certainty. As a practical matter, however, the amount of primer added will generally be in molar excess over the amount of complementary strand (template) when the sequence to be amplified is contained in a mixture of complicated long-chain nucleic acid strands. A large molar excess is preferred to improve the efficiency of the process.

Nucleoside triphosphates, preferably dATP, dCTP, dGTP, dTTP and/or dUTP are also added to the synthesis mixture in adequate amounts. The preferred molarity of nucleotides is as follows 0.025 mM to 1 mM, preferred 0,05 to 0.6 mM, most prefered 0.1 to 0.5 mM.

It is preferred that the polymerase according to the invention is selected from the group of genera of *Thermus, Aquifex, Thermotoga, Thermocridis, Hydrogenobacter, Thermosynchecoccus* and *Thermoanaerobacter.*

It is preferred that the polymerase according to the invention is selected from the group of organisms of *Aquifex aeolicus, Aquifex pyogenes, Thermus thermophilus, Thermus aquaticus, Thermotoga neapolitana, Thermus pacificus, Thermus eggertssonii,* and *Thermotoga maritima.*

It is most preferred that the polymerase is Taq polymerase. However, as will be outlined in more detail below, in some embodiments it is preferred that the polymerase carries a 5'-3' exonuclease activity. In other embodiments it is preferred that the polymerase lacks a 5'-3' exonuclease activity. In most embodiments it is preferred that the polymerase lacks a 3'-5' exonuclease activity.

In one embodiment uracil residues are incorporated during the PCR reaction. Uracil DNA glycosylase (uracil-N-glycosylase) is the product of the *Escherichia coli* ung-gene, and has been cloned, sequenced and expressed in *E. coli.* Uracil DNA glycosylase (UDG) removes these uracil residues from DNA (single- and double-stranded) without destroying the DNA sugar-phosphodiester backbone, thus preventing its use as a hybridization target or as a template for DNA polymerases. The resulting abasic sites are susceptible to hydrolytic cleavage at elevated temperatures. Thus, removal of uracil bases is usually accompanied by fragmentation of the DNA. The person skilled in the art knows how to use the Uracil DNA glycosylase in order to avoid contamination. Likewise both the enzyme as well as the uracil nucleotide may be in the kit according to the invention.

Ideally, the labels of the probes in the first and second or further probe set are fluorescent labels and have an emission wavelength that is very similar. Ideally, that means they may be detected without altering the wavelength adjustment that may be detected by the detection device. It is preferred that the labels of the probes in the first, second and third or further probe set are identical.

It is preferred that the probes carrying the same label differ in melting temperature (Tₘ) in a way that they are distinguishable by melting point on a given instrument, typically harboring a difference in melting temperature of more than 0.1°C, 0.2°C, 0.3°C, 0.4°C, 0.5°C, 1°C, 1.5°C, 2°C, 2.5°C, 3°C, 4°C, 5°C, 6°C, 7°C, 8°C, 9°C, or 10°C. More than 1°C, 1.5°C, 2°C, 2.5°C, 3°C, 4°C, 5°C is preferred.

In one embodiment the melting transitions of the double stranded segments can be determined by monitoring fluorescence intensity of double stranded nucleic acid-specific (dsNAS) dyes. In one embodiment, the double stranded nucleic acid-specific dye is selected from the group consisting of SYBR® Green I, SYBR® Gold, ethidium bromide, propidium bomide, Pico Green, Hoechst 33258, YO-PRO-I and YO-YO-I, SYTO®9, LC Green®, LC Green® Plus+, EvaGreen™. These saturation dyes are capable of existing at sufficiently saturating conditions with respect to the DNA during or after amplification, while minimizing the inhibition of PCR. For example, at maximum PCR-compatible concentrations, the dsDNA binding dye has a percent saturation of at least 50%. In other embodiments, the percent saturation is at least 80%. In yet other embodiments, the percent saturation is at least 99%. It is understood that the percent saturation is the percent fluorescence compared to fluorescence of the same dye at saturating concentrations. Saturating concentration is the concentration that provides the highest fluorescence intensity possible in the presence of a predetermined amount of dsDNA. Because these dyes can be present at significantly higher concentrations without significantly interfering with certain nucleic acid reactions, these dyes may be particularly useful for monitoring the conformation of single-stranded nucleic acids and dsDNA.

The preferred reaction is a polymerase chain reaction.

It is preferred that the probes are selected from the group of TaqMan probe, molecular beacon probe, scorpion probe and light cycler probe. Detection of the amplification product *per* se may be accomplished by using one of the following probes, TaqMan probe, molecular beacon probe, scorpion probe,, light cycler probe, hybridization probe, displacement probe and other types of sequence specific probe formats.

The TaqMan® Assay utilizes the 5' nuclease activity of Taq DNA polymerase to cleave a fluorescently labeled probe (FAM^{™}-Iabeled MGB).

Molecular beacons are single-stranded oligonucleotide hybridization probes that form a stem-and-loop structure (Fig. 2). The loop contains a probe sequence that is complementary to a target sequence, and the stem is formed by the annealing of complementary arm sequences that are located on either side of the probe sequence. A fluorophore is covalently linked to the end of one arm and a quencher is covalently linked to the end of the other arm. Molecular beacons do not fluoresce when they are free in solution. However, when they hybridize to a nucleic acid strand containing a target sequence they undergo a conformational change that enables them to fluoresce brightly. In the absence of targets, the probe is dark, because the stem places the fluorophore so close to the non-fluorescent quencher that they transiently share electrons, eliminating the ability of the fluorophore to fluoresce. When the probe encounters a target molecule, it forms a probe-target hybrid that is longer and more stable than the stem hybrid. The rigidity and length of the probe-target hybrid precludes the simultaneous existence of the stem hybrid. Consequently, the molecular beacon undergoes a spontaneous conformational reorganization that forces the stem hybrid to dissociate and the fluorophore and the quencher to move away from each other, restoring fluorescence. Molecular beacons are added to the assay mixture before carrying out gene amplification and fluorescence is measured in real time. Molecular beacons can be synthesized that possess differently colored fluorophores, enabling the method according to the invention.

The color of the resulting fluorescence, if any, identifies the pathogenic agent in combination with the determination of the melting temperature.

Scorpion primers (Fig: 3) are bi-functional molecules in which a primer is covalently linked to the probe. The molecules also contain a fluorophore and a quencher. In the absence of the target, the quencher nearly absorbs the fluorescence emitted by the fluorophore. During the Scorpion PCR reaction, in the presence of the target, the fluorophore and the quencher separate which leads to an increase in the fluorescence emitted. The fluorescence can be detected and measured in the reaction tube.

A light cycler FRET probe system is a pair of single-stranded fluorescently-labeled oligonucleotides. Probe 1 (the donor probe) is labeled at its 3'end with a donor fluorophore (generally fluorescein) and Probe 2 (the acceptor probe) is labeled at its 5'end with one of four available fluorophores (red 610, 640, 670 or 705). The free 3' hydroxyl group of Probe 2 must be blocked with a phosphate group (P) to prevent Taq DNA polymerase extension. To avoid any steric problems between the donor and the acceptor fluorophores on both probes, there should be a spacer of 1 to 5 nt (4 to 25A distance) to separate the two probes from each other. Before any real-time quantitative PCR reaction takes place, fluorescence background may be observed inside the tube.

During the annealing step of real-time quantitative PCR, the PCR primers and the light cycler probes hybridize to their specific target regions causing the donor dye to come into close proximity to the acceptor dye. When the donor dye is excited by light from the light cycler instrument (hγ1), energy is transferred by Fluorescence Resonance Energy Transfer (FRET) from the donor to the acceptor dye. The energy transfer causes the acceptor dye to emit light (hγ2) at a longer wavelength than the light emitted from the instrument (hγ1). The acceptor fluorophore's emission wavelength is detected by the instrument's optical unit. The increase in measured fluorescent signal is directly proportional to the amount of accumulating target DNA.

Other alternative probes are Eclipse Probes (Epoch, Nanogen), displacement probes (Cheng et al., Nucleic Acids Research, 2004, Vol. 32, No. 7), pleiades probes (NAR 2007 Vol 35 5 e30) and plexor systems (Promega). Of course other probe systems are likewise encompassed by the invention.

In one embodiment of the invention the TaqMan probe is combined with a intercalating dye used for the melting point analysis.

In another embodiment the TaqMan probe is combined with a hybridization probe used for the melting point analysis in a special embodiment the TaqMan probe is the hybridization probe. In another embodiment the TaqMan probe is not the hybridization probe but a separate oligonucleotide serves as hybridization probe.

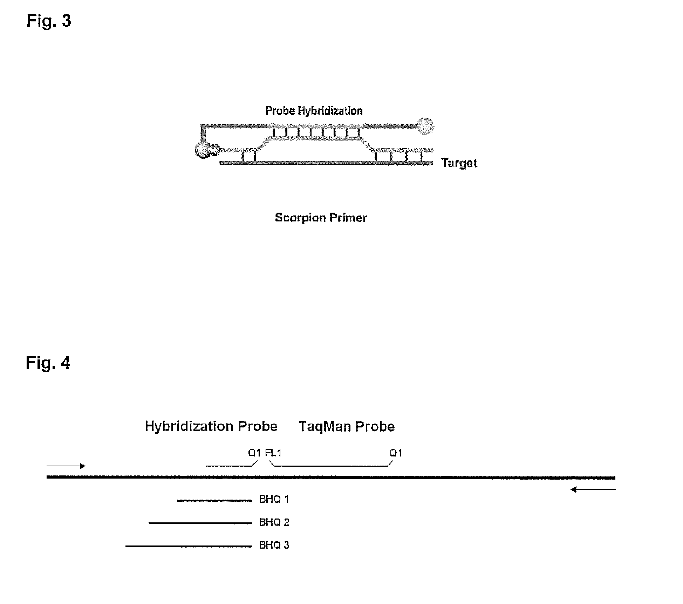

Figure 4 shows a very preferred embodiment. Here, the reaction comprises both a hybridization probe as well as a TaqMan probe. Here, (a) each probe set would, e.g. consists of at least two probes, wherein the probes are the hybridization probes, (b) each of the probes is specific for a nucleic acid sequence, (c) each of the hybridization probes (for example BHQ 1, BHQ 2, BHQ 3 in Fig. 4) in a given probe set carries a different label (see for example column A in Fig. 1), (d) all of the probes in a given probe set have a similar, preferably identical melting temperature (Tₘ) (see for example column A in Fig. 1) when they are dissociated from their target nucleic acid sequence by heating. The amplification of the nucleic acid sequences in the reaction is performed, the amplified nucleic acids are detected and the temperature at which each given labeled probe dissociates from the nucleic acid sequence to which it has bound is determined. In this embodiment it is preferred, although not essentially required, that the melting point of the hybridization probe is lower than the melting point of the primers used for amplification.

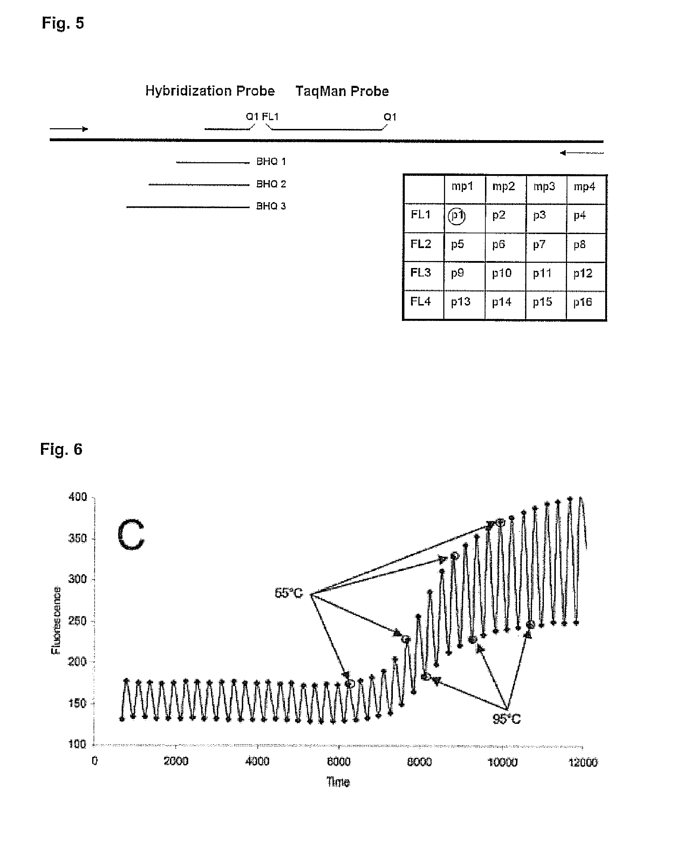

One example is given in Fig. 5. During the PCR reaction at a given melting point (the melting point for the hybridization probe Q1) the hybridization probe Q1 will dissociate for the DNA strand. The hybridization probe carries a quencher. Once dissociated the downstream TaqMan probe will give rise to a signal coming from the fluorescent label which is now no longer quenched. The melting point is known due to the signal (melting point mp1). The label is known (FL1). Hence, it is possible to determine that this hybridization probe was specific for, e.g. pathogen 1 (p1) which is thus known to be present in the reaction. At the same time the TaqMan probe allows on line quantification during the PCR reaction.

It is preferred that the reaction additionally comprises a double strand nucleic acid specific dye. If a double strand specific dye is used it is preferred that this dye is selected from the group of SYBR^{®} Green I, SYBR^{®} Gold, ethidium bromide, propidium bromide, Pico Green, Hoechst 33258, YO-PRO-I and YO-YO-I. SYBR^{®} Green I is very preferred.

According to the invention ideally, the double strand nucleic acid specific dye is spectrally distinguishable from the probe labels.

Ideally, the label is a fluorescent label and the label is selected from the group of FAM (5-or 6-carboxyfluorescein), VIC, NED, Fluorescein, FITC, IRD-700/800, CY3, CY5, CY3.5, CY5.5, HEX, TET, TAMRA, JOE, ROX, BODIPY TMR, Oregon Green, Rhodamine Green, Rhodamine Red, Texas Red, Yakima Yellow, Alexa Fluor PET, Biosearch Blue™, Marina Blue^{®}, Bothell Blue^{®}, Alexa Fluor^{®}. 350 FAM™, SYBR^{®} Green 1, Fluorescein, EvaGreen™, Alexa Fluor^{®} 488 JOE™, VIC™, HEX™, TET™, CAL Fluor^{®} Gold 540, Yakima Yellow^{®}, ROX™, CAL Fluor^{®} Red 610, Cy3.5™, Texas Red^{®}, Alexa Fluor^{®}, 568 Cy5™, Quasar™ 670, LightCycler Red640^{®}, Alexa Fluor 633 Quasar™ 705, LightCycler Red705^{®}, Alexa Fluor^{®} 680, SYTO^{®}9, LC Green^{®}, LC Green^{®} Plus+, EvaGreen™.

The method is alternatively based on the basic principle that melting curve analyses is performed at the end of a PCR reaction with a single dual-labeled probe allows differentiation of targets. To avoid hydrolysis of this probe, as occurs classically during the elongation steps in TaqMan real-time PCR, the melting point (Tₘ) of the probe was chosen to be 10 °C below the Tₘs of the PCR primers. At the end of the PCR reaction, the probe is allowed to hybridize and the mixture is subjected to stepwise increase in temperature, with fluorescence monitored continuously. As in classic Taq-Man real-time PCR, generation of the fluorescence signal by the probe is based on the Förster resonance energy transfer (FRET) phenomenon.

However, and contrary to what happens in TaqMan real-time PCR, no or only partial hydrolosis of the available probe molecules by Taq polymerase is involved in this embodiment. Rather, the procedure relies on the decrease in FRET observed when the probe detaches from its target to achieve a random single-stranded conformation. The mean distance between the reporter and the quencher molecules of the dual-labeled probe will become shorter when the probe is released from its hybrid with the target sequence. Because the FRET effect is inversely proportional to the sixth power of this distance, a difference in fluorescence emission will be readily detectable between hybridized and melted configurations of the probe. A general scheme is shown in Fig. 6. Here, the change in fluorescence is shown cycle by cycle for different reaction temperatures. In a preferred embodiment of this method the polymerase used lacks a 5'-3' exonuclease activity.

In a preferred embodiment, asymmetric primer concentrations are used. This is done by altering the ratio of both primers for each target in a way that the primer generating the DNA strand binding the probe is added at higher concentration than the other primer.

In one alternative embodiment of the method according to the invention, (a) the labeled probe is a group consisting of a hybridization probe and TaqMan probe, (b) the TaqMan probe carries the said label, (c) the said label is a fluorescent label, (d) the TaqMan probe additionally comprises a quencher, (e) optionally the hybridization probe carries an additional quencher that is able to quench the fluorescence of the label attached to the TaqMan probe, (f) said TaqMan probe and said hybridization probe are able to bind said nucleic acid sequence adjacently in such a way that when both probes are bound to their respective sequences, the quencher present on the hybridization probe, at least partially, quenches the fluorescence of the said label on said TaqMan probe. It is however preferred that the hybridization probe carries an additional quencher that is able to quench the fluorescence of the label attached to the TaqMan probe.

In this embodiment the probes with identical labels form a group with hybridization probes which differ in melting temperature (Tm) so that they may be distinguished, e.g. by at least 0.1 °C, 0.5°C, 1°C, 1.5°C, 2°C, 2.5°C, 3°C, 4°C, 5°C, 6°C, 7°C, 8°C, 9°C, or 10°C. More than 0.5°C, 1°C, 1.5°C, 2°C, 2.5°C, 3°C, 4°C, 5°C is preferred. Most preferred is about 5°C. In this embodiment it is preferred also that the melting temperature (Tₘ) of the hybridization probe is lower than the melting temperature (Tₘ) of the TaqMan probe. Hence, once in a reaction, upon annealing, the temperature is elevated, the hybridization probe will dissociate from its complementary strand, generating a first signal, when the Tₘ is reached. The polymerase will then gain access to the TaqMan probe which will result in a second TaqMan signal.

It is also preferred in this embodiment that the hybridization probes have a melting temperature (Tₘ) that is below the temperature at which the polymerase exhibits its optimal activity. This provides for that the hybridization probes dissociate and gives the way free for the polymerase to the TaqMan probe. It is obvious that the method ideally makes use of a polymerase that exhibits a 5'-3' exonuclease activity.

Real-time PCR requires an instrumentation platform that consists of a thermal cycler, a computer, optics for fluorescence excitation and emission collection, and data acquisition and analysis software. These machines, available from several manufacturers, differ in sample capacity (some are 96-well or 384-well standard plate format, others process fewer samples or require specialized glass capillary tubes, some have block format, others a carousel), method of excitation (some use lasers, others broad spectrum light sources with tunable filters or one or more diodes), detection (some use a camera, others a photo multiplier tube, or types of light detection system) and overall sensitivity. There are also platform-specific differences in how the software processes data. In principle the available machines harboring two or more detection channels are suited for the method according to the invention.

The invention relates to a kit comprising at least 6 probes which are able to hybridize, under stringent conditions, to one or more nucleic acid molecules, wherein a) a first group of at least three probes carries a first label and all the probes in this group differ with respect to their melting temperature and b) a second group of at least three probes carries a second label and all the probes in this group differ with respect to their melting temperature

The invention also relates to a kit comprising at least 9 probes which are able to hybridize, under stringent conditions, to one or more nucleic acid molecules, wherein (a) a first group of at least two probes carries a first label and all the probes in this group differ with respect to their melting temperature, (b) a second group of at least two probes carries a second label and (c) a third group of at least two probes carries a third label, and all the probes in this group differ with respect to their melting temperature and at least a third group of at least two probes carries a third label and all the probes in this group differ with respect to their melting temperature. Of course the invention also relates to a kit with more probe sets and/or more probes.

The probes that differ with respect to their melting temperature (Tₘ) differ by at least 0.5°C, 1°C, 1.5°C, 2°C, 2.5°C, 3°C, 4°C, 5°C, 6°C, 7°C, 8°C, 9°C, or 10°C. More than 1°C, 1.5°C, 2°C, 2.5°C, 3°C, 4°C, 5°C is preferred. Ideally the probes differ by about 5°C. Ideally, the probes that have the same label have fixed intervals of difference in melting temperature (Tₘ), selected in a way that they are reliably distinguishable by melting analysis, for example such as 5°C. In such an embodiment the probes that are, for example, fluorescein labeled would have a melting temperature (Tₘ) of, e.g. 40°C, 45°C, 50°C and 55°C.

The kit according to the invention can additionally comprise a buffer, nucleotides and one or enzymes, such as a polymerase.

The kit may be adapted as a premix, wherein the user only needs to add the probe.

Primers and probes according to the invention may be specific for various targets, such as disease markers, pathogens, forensic markers or any other target that may be addressed by means of amplification. The invention is particularly suited for the analysis of pathogens. The most common bacterial disease is tuberculosis, caused by the bacterium *Mycobacterium tuberculosis,* which kills about 2 million people a year, mostly in sub-Saharan Africa. Pathogenic bacteria contribute to other globally important diseases, such as pneumonia, which can be caused by bacteria such as *Streptococcus* and *Pseudomonas,* and foodborne illnesses, which can be caused by bacteria such as *Shigella, Campylobacter* and *Salmonella.* Pathogenic bacteria also cause infections such as tetanus, typhoid fever, diphtheria, syphilis and leprosy. One of the primary pathways by which food or water become contaminated is from the release of untreated sewage into a drinking water supply or onto cropland, with the result that people who eat or drink contaminated sources become infected. In developing countries most sewage is discharged into the environment or on cropland. This is the typical mode of transmission for the infectious agents of cholera, hepatitis A, polio and rotavirus. Thus, in embodiment the primers and probes are specific for one or more pathogenic bacteria.

The kit may used for human or veterinary diagnosis, for testing food or water, for forensic applications or for scientific purposes.

### FIGURE CAPTIONS

**Figure 1**
   Figure 1 shows the principle of the invention. The reactions performed with the probes in, e.g. row one all share a common label. However, the melting temperature of the probes differs. It is thus possible to identify each probe by means of the differing melting temperatures. The probes in column D for example all have the same melting temperature but a different label. It is thus possible to identify each probe by means of the different label.
**Figure 2**
   Figure 2 shows the principle of a molecular beacon probe. Molecular beacons can be used as amplicon detector probes in for example diagnostic assays. Because non-hybridized molecular beacons are dark, it is not necessary to isolate the probe-target hybrids to determine the number of amplicons synthesized during an assay. The are thus ideally suited for the present invention. Molecular beacons are added to the assay mixture before carrying out gene amplification and fluorescence is measured in real time.
**Figure 3**
   With Scorpion probes, sequence-specific priming and PCR product detection is achieved using a single oligonucleotide. The Scorpion probe maintains a stem-loop configuration in the unhybridized state. The fluorophore is attached to the 5' end and is quenched by a moiety coupled to the 3' end. The 3' portion of the stem also contains a sequence that is complementary to the extension product of the primer. This sequence is linked to the 5' end of a specific primer via a non-amplifiable monomer. After extension of the Scorpion primer, the specific probe sequence is able to bind to its complement within the extended amplicon thus opening up the hairpin loop. This prevents the fluorescence from being quenched and a signal is observed.
**Figure 4**
   Figure shows one preferred embodiment of the invention. Here two probes are present in the reaction for each target sequence. A first hybridization probe is present that carries a label, e.g. a fluorescent label that is quenched by an adjacent TaqMan probe. When the melting temperature is reached the hybridization probe dissociates for the strand to which it is bound. The fluorescent label is now no longer quenched and a signal is produced.
**Figure 5**
   Figure shows one preferred embodiment of the invention. A fluorescent signal FL 1 at a melting temperature mp1 is indicative of the presence of the target sequence p1 in the reaction.
**Figure 6**
   Figure 6 shows the intensity of a fluorescent signal with a single dual-labeled probe as it changes throughout a cycle as well as throughout the PCR. The signal is strong when it is bound to the target. It is weaker when dissociated. Also the signal gets stronger towards the end of the reaction due to the increase in template amount.

## Claims

1. Method for simultaneously amplifying and detecting nucleic acid sequences in a reaction comprising the following steps:
(i) providing a sample comprising at least one nucleic acid molecule;
(ii) providing reagents for performing an amplification reaction, wherein the reagents comprise at least two probe sets, wherein
a. each probe set consists of at least three probes;
b. each of the probes is specific for a nucleic acid sequence;
c. each of the probes in a given probe set carries a different fluorescent reporter label;
d. all of the probes in a given probe set have an identical melting temperature (Tₘ) when they are dissociated from their target nucleic acid sequence by heating,
(iii) amplifying the nucleic acid sequences in the reaction;
(iv) detecting the amplified nucleic acids by determining whether the labeled probe has bound its nucleic acid sequence;
(v) detecting the temperature at which each given labeled probe dissociates from the nucleic acid sequence to which it has bound,
wherein the probes carrying the same label differ in melting temperature (Tₘ) in a way that they are distinguishable by melting point, and
wherein the probes are dual-labeled probes comprising said reporter label and a quencher molecule such that a difference in fluorescence emission is detectable between hybridized and melted configurations of said probes.

2. Method of claim 1, wherein the labels of the probes in the first and second probe set are fluorescent labels and have an emission wavelength that is very similar.

3. Method of claim 1 or 2, wherein the amplification reaction is polymerase chain reaction amplification.

4. Method of claim 1 to 3, wherein the reaction additionally comprises a double strand nucleic acid specific dye.

5. Method of claim 4, wherein the double strand nucleic acid specific dye is spectrally distinguishable from the probe labels.

6. Method of claim 1 to 5, wherein the double strand nucleic acid specific dye is selected from the group of SYBR^{®} Green I, SYBR^{®} Gold, ethidium bromide, propidium bromide, Pico Green, Hoechst 33258, YO-PRO-I and YO-YO-I, Boxto, Evagreen, LC Green, LC Green Plus and Syto 9.

7. Method according to any of the above claims, wherein the label is a fluorescent label and the label is selected from the group of FAM (5-or 6-carboxyfluorescein), VIC, NED, Fluorescein, FITC, IRD-700/800, CY3, CY5, CY3.5, CY5.5, HEX, TET, TAMRA, JOE, ROX, BOD1PY TMR, Oregon Green, Rhodamine Green, Rhodamine Red, Texas Red, Yakima Yellow, Alexa Fluor PET, Biosearch Blue™, Marina Blue^{®}, Bothell Blue^{®}, Alexa Fluor^{®}, 350 FAM™, SYBR^{®} Green 1, Fluorescein, EvaGreen™, Alexa Fluor^{®} 488 JOE™, VIC™, HEX™, TET™, CAL Fluor^{®} Gold 540, Yakima Yellow^{®}, ROX™, CAL Fluor^{®}, Red 610, Cy3.5™, Texas Red^{®}, Alexa Fluor^{®}, 568 Cy5™, Quasar™ 670, LightCycler Red640^{®}, Alexa Fluor 633 Quasar™ 705, LightCycler Red705^{®}, Alexa Fluor^{®} 680, SYTO^{®}9, LC Green^{®}, LC Green^{®} Plus+, EvaGreen™.

8. Method according to any of the above claims, wherein the dual-labeled probes are Molecular Beacon probes or TaqMan probes.

9. Kit comprising at least 6 dual-labeled probes which are able to hybridize, under stringent conditions, to one or more nucleic acid molecules, wherein
a) a first group of at least three probes carries a first label and all the probes in this group differ with respect to their melting temperature and
b) a second group of at least three probes carries a second label and all the probes in this group differ with respect to their melting temperature, wherein the dual-labeled probes comprise said reporter label and a quencher molecule such that a difference in fluorescence emission is detectable between hybridized and melted configurations of said probes.

10. The kit according to claim 9, wherein the dual-labeled probes are Molecular Beacon probes or TaqMan probes.
